# EUROPEAN PATENT APPLICATION

(11) **EP 3 299 024 A1**
(43) Date of publication of application: **28.03.2018**
(21) Application number: 16796508.6
(22) Date of filing: 17.05.2016
(51) Int. Cl.: A61K 35/644, A23L 21/20, A23L 33/10, A61P 13/00

(54) **URINARY SYSTEM SYMPTOM AMELIORATION AGENT**

(30) Priority: 20.05.2015 JP 2015102783
(71) Applicant: Yamada Bee Company, Inc., Tomata-gun, Okayama 708-0393 (JP)
(72) Inventor: ASAMA Takashi, Tomata-gun Okayama 708-0393 (JP); FUKUSHIMA Shinobu, Tomata-gun Okayama 708-0393 (JP)
(74) Representative: Grünecker Patent- und Rechtsanwälte PartG mbB
(86) International application number: PCT/JP2016/064642
(87) International publication number: WO 2016/186115

(57) **Abstract**

The present invention provides a urinary organ symptoms improving agent comprising propolis or a processed product thereof as an active ingredient.

## Description

### Technical Field

The present invention relates to a urinary organ symptoms improving agent.

### Background Art

Propolis is known to have many bioactivities such as antibacterial activities and antitumor activities (for example, Patent Literatures 1 to 4).

### Citation List

### Patent Literature

Patent Literature 1: JP2005-22994A
Patent Literature 2: JP2005-60242A
Patent Literature 3: JP2007-131592A
Patent Literature 4: JP2008-214235A

### Summary of Invention

### Technical Problem

An object of the present invention is to provide a novel use of propolis.

### Solution to Problem

The present inventors have found that propolis has an effect of improving urinary organ symptoms. The present invention is based on this novel finding.

Specifically, the present invention provides a urinary organ symptoms improving agent comprising propolis or a processed product thereof as an active ingredient.

The urinary organ symptoms improving agent of the present invention can be used as a food composition or pharmaceutical product for improving urinary organ symptoms. Examples of the food composition include foods, health foods, foods with function claims (functional foods), nutritional supplementary foods, supplements, and foods for specified health use. Propolis has been conventionally used as foods and the like, and its safety in living organisms has been established. Therefore, the food composition or pharmaceutical product for improving urinary organ symptoms may be taken continuously over a long period of time.

Another aspect of the present invention is propolis or a processed product thereof for use in improving urinary organ symptoms. A further aspect of the present invention is an agent comprising propolis or a processed product thereof as an active ingredient for use in improving urinary organ symptoms.

A still further aspect of the present invention is use of propolis or a processed product thereof in manufacturing a urinary organ symptoms improving agent.

A yet further aspect of the present invention is a method of improving urinary organ symptoms comprising administering an effective amount of propolis or a processed product thereof to a subject in need thereof.

### Advantageous Effect of Invention

According to the present invention, there is provided a urinary organ symptoms improving agent comprising propolis or a processed product thereof as an active ingredient.

### Description of Embodiments

The embodiments of the present invention will be illustrated in detail as follows. However, the present invention is not limited to the following embodiments.

The urinary organ symptoms improving agent of the present invention comprises propolis or a processed product thereof as an active ingredient.

Propolis is a resinous or waxy substance constituting the nest wall of a honeycomb. Propolis is known to contain cinnamic acid derivatives, flavonoids, vitamins, minerals, and the like. Propolis may be derived from any origin such as Brazilian, Chinese, European, Oceanian, or American origin. In addition, propolis may be derived from any plant such as alecrim and Eucalyptus. Among them, Brazilian propolis is preferred, because it is derived from Baccharis dracunculifolia of Baccharis genus in the family Asteraceae and contains a large amount of cinnamic acid derivatives such as artepillin C. Also, propolis may be in any rank such as Super Green and Ultra Green Propolis. Examples of the species of honey bee used for gathering propolis include, but are not limited to, the Western honey bee (Apis mellifera) and the Africanized honey bee. Among them, the Africanized honey bee (a hybrid of the African honey bee (A. mellifera scutellata) with the Western honey bee), which produces propolis of Brazilian origin, is preferred. For example, propolis can be obtained as a beekeeping product according to any conventional method.

The propolis may be raw propolis mass, or a processed product of propolis which is obtained by subjecting the raw propolis mass to processing such as grinding, supercritical extraction, extraction with water or a hydrophilic organic solvent, concentration or pulverization of the propolis extract, and granulation of the powder. Among them, a hydrophilic organic solvent extract obtained by extraction of propolis with a hydrophilic organic solvent is preferred because it contains the active components of propolis which has been extracted efficiently with a good balance in a short time. The hydrophilic organic solvent used in extraction is preferably ethanol.

As the propolis or processed product thereof, a commercially available product may be used. Concrete examples of the propolis or processed product thereof include Propolis 300, Propolis liquid 30 (Brazilian origin), Propolis Help, Propolis Grain, Propolis Granule A.P.C, Propolis Mild, and Propolis Drink, all produced and marketed by Yamada Bee Company, Inc.

The above-mentioned raw propolis mass or processed product thereof may be used in single form or combination form of two or more thereof to prepare the urinary organ symptoms improving agent of the present invention.

The urinary organ symptoms improving agent of the present invention comprises propolis or a processed product thereof as an active ingredient and can thereby improve urinary organ symptoms, that is, for example, inhibit the onset of urinary organ symptoms, reduce the incidence of urinary organ symptoms, and alleviate the urinary organ symptoms.

Examples of the urinary organ symptoms include urination disorders such as high frequency of going to toilet (pollakiuria), feeling residual urine (residual urine), and inability to urinate without strain (dysuria) as well as feeling some discomfort with urination. The urinary organ symptoms improving agent of the present invention can significantly improve urination disorders such as pollakiuria, residual urine, and dysuria, and can therefore be suitably used as an agent for improving urination disorder (preferably a pollakiuria improving agent, a residual urine improving agent, and/or a dysuria improving agent).

The urinary organ symptoms improving agent of the present invention may comprise only the active ingredient, propolis or a processed product thereof, but may further comprise other ingredients unless they interfere with the effect of the present invention. Examples of the other ingredients include pharmaceutically acceptable ingredients (such as excipients, binders, lubricants, disintegrants, emulsifiers, surfactants, bases, solubilizers, and suspending agents), and ingredients acceptable as food (such as minerals, vitamins, flavonoids, quinones, polyphenols, amino acids, nucleic acids, essential fatty acids, refreshing agents, binders, sweeteners, disintegrants, lubricants, colorants, flavors, stabilizers, preservatives, sustained release regulators, surfactants, solubilizers, and wetting agents).

The urinary organ symptoms improving agent of the present invention can be used at the dose of not less than 1 mg and not more than 10 g per day for an adult with a body weight of 60 kg in terms of the amount of the active ingredient, preferably not less than 50 mg and not more than 8 g, more preferably not less than 150 mg and not more than 3 g, and further more preferably not less than 150 mg and not more than 1.5 g. The dose of the urinary organ symptoms improving agent can be appropriately set in the above-mentioned range depending on such factors as physical conditions of the subject receiving it, the mode of administration, and its combination with other agents.

The urinary organ symptoms improving agent of the present invention may be administrated orally (ingested) or administered parenterally. The vertigo alleviating agent of the present invention may be administered once a day or administered in divided doses multiple times a day such as twice a day and three times a day, so long as the amount of the active ingredient per day is in the above-mentioned range.

Because the urinary organ symptoms improving agent of the present invention exhibits the effect of improving urinary organ symptoms more remarkably, it is preferable that the agent is used to administer it orally once a day for an adult with a body weight of 60 kg in the amount of not less than 150 mg and not more than 1.5 g in terms of the amount of the active ingredient.

The urinary organ symptoms improving agent of the present invention may be in any form such as solid, liquid, and paste. The dosage form of the urinary organ symptoms improving agent of the present invention may be, for example, uncoated tablets, sugar coated tablets, granules, powders, tablets, and capsules (hard capsules, soft capsules, seamless capsules). The urinary organ symptoms improving agent of the present invention can be prepared, for example, by forming propolis or a processed product thereof as an active ingredient into the above-mentioned dosage form as needed after as mixing it with other ingredients.

The urinary organ symptoms improving agent of the present invention may be used as pharmaceutical products, quasi pharmaceutical products, and food compositions in themselves, and used by addition thereof to pharmaceutical products, quasi pharmaceutical products, and food compositions. It is preferable that the food compositions are enhanced in the third-order function of food (a function of adjusting physical conditions, such as a function of improving urinary organ symptoms). Examples of the food composition having enhanced third-order function of food include health foods, foods with function claims (functional foods), nutritional supplementary foods, supplements, and foods for specified health use.

The pharmaceutical products, quasi pharmaceutical products, or food compositions consisting of the urinary organ symptoms improving agent of the present invention, or the pharmaceutical products, quasi pharmaceutical products, or food compositions comprising the urinary organ symptoms improving agent of the present invention may be for use in improving urinary organ symptoms, and may be attached with an indication such as "to improve urinary organ symptoms".

The content of the urinary organ symptoms improving agent of the present invention in the pharmaceutical products, quasi pharmaceutical products, or food compositions may be appropriately set depending on the types of pharmaceutical products, quasi pharmaceutical products, or food compositions, and the like, so that the amount of the active ingredient given per day is in the above-mentioned range.

When using the urinary organ symptoms improving agent of the present invention as a food composition in itself or using it by adding it to a food composition, examples of the form of the food composition include, but is not particularly limited to, beverages (such as soft drinks such as coffee, juice, tea drinks and jelly drinks, milk drinks, lactobacillus beverages, yoghurt drinks, and carbonated drinks), spreads (such as custard creams), pastes (such as fruit pastes), Western confectioneries (such as chocolates, donuts, pies, cream puffs, gums, jellies, candies, cookies, cakes, and puddings), Japanese sweets (such as Daifuku, Mochi (rice cake), Manju, Castella, Anmitsu, and Yokan), frozen desserts (such as ice creams, ice candies, and sherbets), food products (such as curries, beef bowls, Zosui (Japanese rice soup), miso soups, soups, meat sauces, pastas, pickles, and jams), and seasonings (such as dressings, Frikake (dry Japanese seasoning), umami seasonings, and soup mixes).

When using the urinary organ symptoms improving agent of the present invention as a food composition having enhanced third-order function of food (such as a health food, a food with a function claim (functional food), a nutritional supplementary food, supplement, and a food for specified health use) in itself or using by adding it to the food composition, examples of the form of the food having enhanced third-order function of food include uncoated tablets, sugar coated tablets, granules, powders, tablets, and capsules (hard capsules, soft capsules, seamless capsules) in addition to the above-mentioned form of food.

When using the urinary organ symptoms improving agent of the present invention as a pharmaceutical product or quasi pharmaceutical product in itself or using by adding it to a pharmaceutical product or quasi pharmaceutical product, examples of the form of the pharmaceutical product or quasi pharmaceutical product include, but is not particularly limited to, uncoated tablets, sugar coated tablets, granules, powders, tablets, and capsules (hard capsules, soft capsules, seamless capsules).

A method for preparing the pharmaceutical product, quasi pharmaceutical product, or food composition having the urinary organ symptoms improving agent of the present invention added is not particularly limited and it can be prepared appropriately according to any method known in the art. For example, the urinary organ symptoms improving agent of the present invention can be mixed with an intermediate product or final product obtained in the process of preparing a pharmaceutical product, quasi pharmaceutical product, or food composition to obtain the pharmaceutical product, quasi pharmaceutical product, or food composition used in the above-mentioned applications.

Another aspect of the present invention mentioned above is propolis or a processed product thereof for use in improving urinary organ symptoms. A further aspect of the present invention is an agent comprising propolis or a processed product thereof as an active ingredient, for use in improving urinary organ symptoms. A still further aspect of the present invention is use of propolis or a processed product thereof in manufacturing a urinary organ symptoms improving agent.

A yet further aspect of the present invention mentioned above is a method of improving urinary organ symptoms comprising administering an effective amount of propolis or a processed product thereof to a subject in need thereof. The effective amount may be not less than 1 mg and not more than 10 g per day for an adult with a body weight of 60 kg, or not less than 50 mg and not more than 8 g, not less than 150 mg and not more than 3 g, or not less than 150 mg and not more than 1.5 g. The subject is preferably a mammal, and more preferably human.

### Example

The present invention will be illustrated more specifically based on the example as follows. However, the present invention is not limited to the following example.

### [Test method]

This test method was carried out by using 150 healthy volunteers as subjects and allowing them to ingest three pills of Propolis 300 (from Yamada Bee Company, Inc.: containing 226.8 mg/3 grains of Brazilian propolis extract) daily for 8 weeks. A questionnaire was carried out to evaluate the severity of urinary organ symptoms before and after the ingestion such as "feeling some discomfort with urination such as high frequency of going to toilet, feeling residual urine, and inability to urinate without strain" with three levels of scores (1: yes; 2: slight; 3; no). From the questionnaire results, a cross-tabulation table was created with "1: yes" and "2: slight" as "yes", and "3: no" as "no". The presence or absence of symptoms before and after the ingestion was analyzed by McNemar test.

The results of analysis is shown in Table 1. The shaded cell indicates that amelioration was observed after 8 weeks of ingestion.

**[Table 1]**

| Before ingestion | After 8 weeks of ingestion | | | Within-group test |
|---|---|---|---|---|
| | Yes | No | Total | |
| Yes | 10 | 16 | 26 | *p* <0.0001 |
| No | 0 | 124 | 124 | |
| Total | 10 | 140 | 150 | |

By the ingestion of propolis, it was observed that the incidence of urinary organ symptoms was reduced or the onset of urinary organ symptoms was inhibited, indicating that urinary organ symptoms were significantly improved.

## Claims

1. A urinary organ symptoms improving agent comprising propolis or a processed product thereof as an active ingredient.

2. A food composition for improving urinary organ symptoms comprising the urinary organ symptoms improving agent according to claim 1.

3. A pharmaceutical product for improving urinary organ symptoms comprising the urinary organ symptoms improving agent according to claim 1.
